# FASCICULE DE BREVET EUROPEEN

(11) **EP 3 675 918 B1**
(45) Date de publication et mention de la délivrance du brevet: **01.11.2023**
(21) Numéro de dépôt: 18768927.8
(22) Date de dépôt: 17.08.2018
(51) Int. Cl.: A61L 9/04

(54) **SYSTEME DE DIFFUSION DE FRAGRANCES**
DUFTABGABESYSTEM
FRAGRANCE DELIVERY SYSTEM

(30) Priorité: 30.08.2017 FR 1757976
(43) Date de publication de la demande: 08.07.2020
(73) Titulaire: Scentys, 75002 Paris (FR)
(72) Inventeur: SUISSA, David, 94120 FONTENAY SOUS BOIS (FR); MARTIN, Laurent, 49610 SAINT-MELAINE-SUR-AUBANCE (FR)
(74) Mandataire: Ipside
(86) Numéro de dépôt international: PCT/FR2018/052073
(87) Numéro de publication internationale: WO 2019/043319

(56) Documents cités:
- EP-A1- 1 932 545
- US-A1- 2017 106 114

## Description

### Domaine de l'invention

La présente invention concerne le domaine de la diffusion de fragrances utilisant des supports solides dans lesquels sont adsorbées des molécules odorantes que l'on libère dans l'atmosphère par diffusion spontanée ou à l'aide d'un flux d'air. Elle concerne en particulier le domaine de la diffusion de fragrances dans un habitacle d'un véhicule (automobile, ferroviaire, avion, bateau,...) mettant en oeuvre des capsules interchangeables comprenant des éléments de substrat dans lesquels est adsorbé un parfum.

Elle concerne plus précisément les systèmes de diffusions passifs qui offrent l'avantage de ne nécessiter aucun apport en énergie. Le parfum est mis à l'air libre et profite des différents mouvements d'air pour se disperser dans l'ambiance à parfumer.

### Etat de la technique

On connaît dans l'état de la technique la demande de brevet européen EP1932545 décrivant un récipient pour diffuser des substances volatiles imprégnées de matières solides ou semi-solides, caractérisé en ce que ledit récipient est rempli d'une charge de granulés desdits matériaux et est pourvu d'ouvertures de forme et de taille permettant la libération de la substance volatile mais pas des granulés.

On connaît aussi la demande de brevet américain US2017/106114 décrivant une poche perméable aux fluides renfermant une chambre, la poche comprenant une liaison qui définit au moins partiellement la chambre ; une pluralité de particules contenues dans la chambre, les particules comprenant un parfum ; dans lequel la poche comprend une boucle ou une ouverture dimensionnée pour s'adapter à un cintre de vêtement.

### Inconvénients de l'art antérieur

Les solutions décrite dans l'art antérieur prévoient le stockage des molécules odorantes dans un matériau support tel qu'un solide poreux ou une gélatine permettant la libération par évaporation. Les molécules parfumées sont des petites molécules ayant une forte tendance à diffuser à travers un grand nombre de matériaux supports, tant poreux que la gélatine. Les moteurs de cette diffusion sont la volatilité et le partage entre la phase intérieure et la phase extérieure. Les pertes liées à la diffusion limitent la stabilité de ces systèmes dans des produits.

Les solutions proposées dans l'art antérieur présentent de ce fait une faible durée de vie, typiquement de quelques jours après le retrait de leur conditionnement étanche.

La vaporisation du parfum se fait de manière constante, que le support soit au repos ou soit animé d'un mouvement oscillant.

Le balancement du récipient produit simplement un déplacement de la masse d'air où se produit cette évaporation, afin d'élargir la zone de perception du produit évaporé. Au repos, l'évaporation se poursuit de manière inchangée, simplement la circulation d'air moindre va conduire à une plus forte concentration à l'intérieur du récipient plutôt qu'une diffusion dans un volume plus important. Il crée un nuage olfactif à la surface du substrat, et dont les constituants sont entraînés par convection naturelle.

De ce fait, la durée de vie de la cartouche est faible : elle correspond au temps nécessaire à l'évaporation du parfum soit quelques jours. Pour compenser ce vieillissement rapide, on pourrait certes augmenter le volume de parfum, mais la diffusion - constante - du parfum ne serait pas maîtrisée et pourrait indisposer en raison de l'intensité et du volume de parfum évaporé.

### Solution apportée par l'invention

L'invention vise à remédier aux inconvénients des différentes solutions de l'art antérieur en proposant un système de diffusion ne nécessitant aucune alimentation électrique, et permettant d'accélérer la diffusion des substances olfactives par rapport à la vitesse de diffusion purement statique, en préservant néanmoins une durée de vie significative, et supérieure à celle observée dans les solutions prévoyant un flux d'air forcé traversant la cartouche.

L'objectif de cette innovation est de créer un dispositif permettant la diffusion efficace de parfum dans des volumes modestes (quelques m³) en ne faisant appel qu'à une énergie mécanique.

L'invention concerne selon son acception la plus générale un système de diffusion de fragrances conforme à la revendication 1, prise isolément et/ou en combinaison avec les caractéristiques d'une ou de plusieurs des revendications dépendantes.

### Description détaillée d'exemples non limitatifs de l'invention

La présente invention sera mieux comprise à la lecture de la description qui suit, se référant aux dessins annexés relatifs à des exemples non limitatifs de réalisation, où :

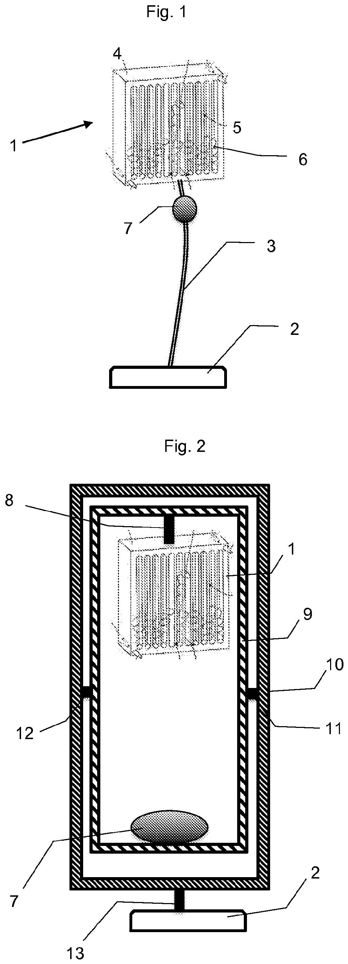

- la figure 1 représente un premier exemple de réalisation
- la figure 2 représente un deuxième exemple de réalisation, qui n'est pas conforme à l'invention

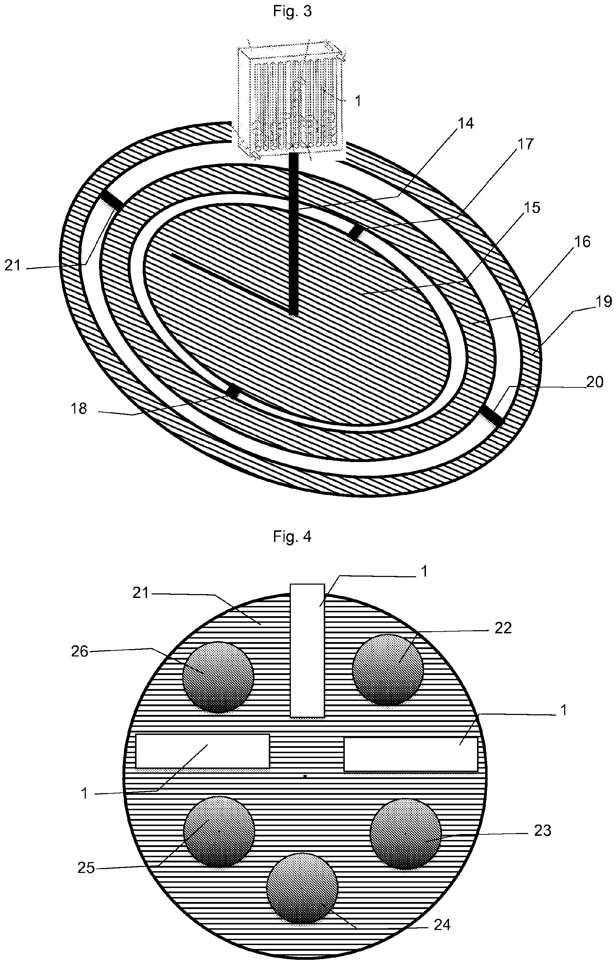

- la figure 3 représente un troisième exemple de réalisation, qui n'est pas conforme à l'invention
- la figure 4 représente un quatrième exemple de réalisation, qui n'est pas conforme à l'invention.

La solution selon l'invention consiste à utiliser un système à énergie entièrement mécanique permettant de créer une qualité de diffusion d'un niveau similaire aux dispositifs électriques, mais n'utilisant aucune source d'énergie électrique extérieure et de fixer les molécules olfactives dans un substrat de type polymère.

### Fixation des molécules olfactives sur le substrat

Les éléments de substrat sont solides. Ils sont réalisés au moins en partie en un matériau polymère. Les polymères sont notamment des élastomères, par exemple un bipolymère de type polyamide et de polyéther block amide. En fonction du matériau polymère utilisé et du parfum, les billes diffusent naturellement les molécules de parfum pendant 6 à 18 mois que de manière très peu perceptible. Un exemple de tel matériau consiste en du Pebax^{®} (nom commercial).

En l'état, les éléments de substrat diffusent spontanément en petite quantité les molécules de parfum qu'elles contiennent par évaporation naturelle, ou plus particulièrement par un phénomène de désorption.

Par contre, lorsque le substrat est soumis à un flux d'air, les interactions mécaniques augmentent notablement, jusqu'à 500 fois, le taux de libération des molécules olfactives.

Les éléments de substrat sont des billes sphéroïdes de matériau polymère et les molécules de parfum sont adsorbées dans tout le volume de chaque bille.

A titre d'exemple, les billes en matériau polymère présentent chacune, avant adsorption du parfum, une plus petite dimension égale à 3 mm et une plus grande dimension égale à 4 mm, et, après adsorption du parfum, une plus petite dimension égale à 4 mm et une plus grande dimension égale à 6 mm. Le poids de parfum adsorbé dans chaque bille correspond sensiblement au poids d'une bille avant adsorption du parfum.

Le parfum est encapsulé dans des perles de polymères avec des liaisons électrostatiques assez fortes entre les molécules composant le parfum et les perles de polymères. Sans ventilation forcée, le mouvement de l'air n'a pas suffisamment de force pour venir arracher les molécules de parfums et la liaison électrostatique l'emporte. Dans cette configuration, la diffusion de parfum nécessite de l'ordre de 18 mois entre le moment où la capsule de parfum est placée à l'air libre en étant complètement pleine en parfum et le moment où tout le parfum disponible a été diffusé ; la diffusion se fait de manière linéaire dans le temps. Une capsule de parfum contenant en moyenne 2g de concentré de parfum, cela signifie que la quantité de parfum relargué en une journée est de : 3,7×10-3 g /jour, soit 1,5×10-4 g/h.

Les perles de polymères utilisées sont un polymère bi composant comportant des parties apolaires et des parties polaires ce qui permet d'avoir une interaction électro-statique relativement forte avec les différentes molécules composant le concentré de parfum qui sont elles-mêmes soit polaires soit apolaires. A noter également qu'y compris quand la température est élevée (par exemple dans un habitacle de voiture, train ou avion laissés en plein soleil), le parfum relargué est en quantité très faible : cela permet ainsi de ne pas parfumer son habitacle de manière trop forte quand l'habitacle est au repos y compris en pleine chaleur.

En ventilation forcée, l'énergie cinétique apportée par le mouvement de l'air est suffisant pour arracher les molécules de parfums et dans ce cadre-là, l'intégralité du concentré de parfum est diffusée dans l'air en environ 24h. La quantité de parfum relargué dans l'air est ainsi de l'ordre de 2 g/jour, soit 0,08 g/h.

A titre de comparaison, une personne qui se parfume avec une eau de toilette de 100ml contenant 10% de concentré de parfum en faisant 3 pulvérisations sur elle, aura mis 0,06g de concentré sur elle (un flacon de 100ml permet en moyenne de faire 500 pulvérisations), soit une quantité de parfum équivalente à celle relarguée par la capsule.

Ainsi au repos, l'intensité olfactive relarguée dans l'air est de l'ordre de 1000 fois moins que ce que relargue une personne qui s'est parfumée, et l'intensité olfactive en ventilation forcée sera du même ordre de grandeur qu'une personne parfumée.

Le principe général mis en oeuvre par les systèmes selon l'invention consiste à utiliser l'énergie cinétique d'une base mobile supportée par une liaison instable, qui peut prendre différentes configurations :
- une tige flexible reliant un support de cartouche contenant une ou plusieurs cartouches, à un socle de fixation fixé sur une surface solide, par exemple la planche de bord ou le plafond d'un véhicule comme une voiture ou un train. Lors des accélérations et décélérations du véhicule tant selon l'axe longitudinal que selon l'axe transversal (lors des changements de direction) ou vertical (passage sur des revêtements inégaux d'un véhicule automobile ou turbulences pour un aéronef), le support de cartouche est soumise à des mouvements oscillant autour d'une position d'équilibre.
- Un pivot autour duquel le support de cartouche contenant une ou plusieurs cartouches est articulé.
   ∘ Ce pivot peut être horizontal, le support de cartouche étant suspendu pour permettre un mouvement de balancement autour d'une position d'équilibre.
   ∘ Ce pivot peut être vertical, le support de cartouche étant libre en rotation pour permettre un mouvement de balancement autour d'une position d'équilibre. Par extrapolation, on entendra au sens du présent brevet par «déplacement oscillant» également une rotation de plusieurs tours autour du pivot. Le mouvement sera favorisé par la présence d'un balourd sur le support. Cette deuxième configuration n'est pas conforme à l'invention.

Les mouvements oscillants du support de cartouche provoquent un déplacement relatif de la cartouche par rapport à l'air environnant, ce qui favorise la libération des substances olfactives, de manière maîtrisée.

La cartouche est de préférence supportée - et non pas suspendue - par l'élément de liaison au support de fixation, et de ce fait est placé au-dessus du support.

Cette configuration assure plus de « nervosité » au dispositif, qui entre en oscillation dès que la cartouche est soumise à un déplacement, résultant par exemple de l'instabilité de la tige flexible et d'une accélération transmise au support. Le déplacement de la cartouche amplifie la déformation flexible de la tige, contrairement à une solution où la cartouche est suspendue à un lien souple où le déplacement aura tendance à compenser l'amplitude du mouvement oscillant.

La récupération mécanique, pour la mise en fonction du dispositif se traduit généralement par un fonctionnement intermittent. Ce mode de fonctionnement intermittent permet d'éviter aux usagers de s'habituer aux odeurs diffusées et de perdre la perception de ces fragrances. L'intermittence permet de réactiver régulièrement la perception de l'odeur en fonction des mouvements et action du dispositif. Cette inconstance, au-delà d'imposer un fonctionnement irrégulier propose plutôt une expérience liée à l'activité du produit.

Le principe des solutions présentées à titre d'exemples non limitatifs est basé sur l'utilisation d'une capsule de parfum sec, telle que décrite dans le brevet WO2013021114. Cette solution assure à l'invention un fonctionnement sûr et pratique pour l'utilisateur.

En particulier l'invention vise à favoriser l'extraction du parfum par une mise en mouvement de la capsule de parfum en lieu et place de la génération du flux d'air par un élément tiers, type ventilateur.

D'un point de vue technique, la performance de la diffusion est liée à l'agitation des molécules d'air autour des billes de parfum contenues dans la capsule. Le débit et la pression d'air autour des billes favorisent la désorption du parfum depuis les billes, et le flux d'air améliore la distribution du parfum dans l'air.

### Description d'un premier mode de réalisation

Selon ce premier exemple de réalisation, le système de diffusion comprend une cartouche (1), un socle (2) et une tige flexible (3).

La cartouche (1) est constituée par une coque (4) dont les faces principales sont formées par des grilles (5). Cette coque renferme des billes (6) solides réalisées en un matériau de substrat propre à autoriser la pénétration ou la fixation au moins à leur périphérie de molécules du parfum.

Cette cartouche (1) est fixée sur une tige flexible (3), par exemple une corde à piano en acier dont l'autre extrémité est fixée sur un socle (2) comportant par exemple à sa surface inférieure une ventouse pour la fixation sur une surface telle qu'un tableau de bord d'un véhicule.

La tige (3) est munie à sa partie supérieure d'une masselotte (7). La masselotte est mise en mouvement par tous les mouvements extérieurs tels que les mouvements et vibrations d'un véhicule et/ou les agitations manuelles. La souplesse de la tige, sa longueur et la masse de la masselotte conditionnent la qualité de la diffusion. Par exemple en voiture, de bons résultat ont été obtenus avec une tige en acier de 0.8 à 1.5mm d'épaisseur, d'une dizaine de cm de long et une masselotte de 50 à 80g environ.

### Description d'un second mode de réalisation

Dans ce deuxième mode de réalisation qui n'est pas conforme à l'invention, la cartouche (1) est supportée par un pivot vertical (8) fixé sur un premier cadre (9) oscillant. Ce premier cadre (9) oscillant est disposé à l'intérieur d'un second cadre (10) par l'intermédiaire de deux axes horizontaux (11,12). L'ensemble des deux cadres (9, 10) forme un cardan, monté pivotant par rapport au socle (2) par l'intermédiaire d'un pivot vertical (13).

Le premier cadre (9) comporte optionnellement une masselotte (7) de manière à ce que l'axe de pivot (8) du balancier soit toujours perpendiculaire au sens des accélérations. Ainsi, quel que soit le sens des variations d'accélération, le disque de pivot va s'aligner et le balancier pourra pivoter librement sur son axe.

### Description d'un troisième mode de réalisation

Selon cette troisième variante qui n'est pas conforme à l'invention, la cartouche (1) est montée sur un disque (15) par l'intermédiaire d'une tige (14), qui peut optionnellement être élastique, ou rigide. Ce disque (15) est monté pivotant par rapport à un premier cadre (16), par l'intermédiaire de deux pivots (17, 18) orientés selon une première direction. Ce cadre est lui-même monté pivotant par rapport à un deuxième cadre (19), par l'intermédiaire de deux pivots (20, 21) orientés perpendiculairement à la première direction.

### Description d'un quatrième mode de réalisation

Selon cette autre variante qui n'est pas conforme à l'invention, la cartouche (1) est supportée par un volant d'inertie constitué par un plateau discal (21), par exemple un volant métallique d'une masse importante est mis en rotation manuelle par l'utilisateur. Guidé en rotation par des éléments à faibles coefficients de friction, une fois la vitesse maximale atteinte, l'inertie va permettre à la masse tournante de conserver son mouvement pendant un bon moment.

Les cartouches (1) sont disposées radialement selon une direction normale à l'axe de rotation, de manière à correctement prendre le vent relatif créé par l'opposition de l'air au mouvement de rotation. Pour fonctionner correctement, ce dispositif doit bénéficier d'un ratio 'Masse en rotation / (Masse des capsules * Frottement)' élevé, de manière à tourner suffisamment longtemps à la vitesse désirée.

L'ensemble est mis en pivot sur un socle fixe avec un système de roulement à bille et est équipé d'une masselotte permettant d'ajouter un balourd significatif. Une ou plusieurs capsules fixées en périphérie du plateau discal sont ainsi animées par le changement d'orientation du dispositif. Ce dispositif s'est révélé efficace sur la planche de bord d'un véhicule, mais semble pouvoir être intégré à un véhicule proposant des variations de vitesses et de direction régulières tels qu'un bus, un train, un tramway ou un métro.

Optionnellement, le plateau (21) est équipé de lests (22 à 26), de manière déséquilibrée pour favoriser la mise en mouvement par les accélérations du véhicule transmises par l'intermédiaire du socle.

## Revendications

1. - Système de diffusion de fragrances comprenant au moins une cartouche (1) contenant des éléments solides chargés par adsorption d'une composition olfactive et s'ouvrant par une grille (5) présentant des orifices de section inférieure à la section desdits éléments solides, **caractérisé en ce que** lesdits éléments solides sont constitués de billes sphéroïdes en matériau polymère, **en ce que** ladite cartouche (1) est montée sur un support de cartouche maintenant la cartouche et reliée à un socle (2) de fixation sur une partie fixe par une liaison instable apte à assurer un déplacement oscillant dudit support de cartouche autour d'une position de référence, **en ce qu'**il présente un moyen de fixation dans l'habitacle d'un véhicule par l'intermédiaire d'un socle (2) de fixation sur une partie fixe d'un véhicule par une liaison instable apte à assurer un déplacement oscillant dudit support de cartouche autour d'une position de référence lors des accélérations de ladite partie fixe du véhicule selon au moins un axe, et **en ce que** ledit support de cartouche est relié au socle par une tige flexible (3) munie d'une masselotte (7) à son extrémité la plus proche de la cartouche (1).

2. - Système de diffusion de fragrances selon la revendication 1 **caractérisé en ce que** ladite cartouche (1) est disposé au-dessus dudit socle (2).

3. - Système de diffusion de fragrances selon la revendication 1 ou 2 **caractérisé en ce que** lesdits déplacements oscillants comportent un vecteur perpendiculaire à la surface de ladite grille (5).

## Patentansprüche

1. Duftverteilungssystem, umfassend mindestens eine Kartusche (1), die feste Element, die durch Adsorption mit olfaktorischen Komponenten beladen sind, enthält und sich durch ein Gitter (5) öffnet, das Löcher mit einem Querschnitt aufweist, der kleiner als der Querschnitt der festen Elemente ist, **dadurch gekennzeichnet, dass** die festen Elemente als kugelförmige Kügelchen aus Polymermaterial ausgebildet sind, dadurch, dass die Kartusche (1) auf einen Kartuschenhalter, der die Kartusche festhält, montiert ist und mit einer Basis (2) zur Befestigung an einem festen Teil durch eine instabile Verbindung verbunden ist, die dazu ausgelegt ist, eine oszillierende Bewegung des Kartuschenhalters um eine Referenzposition zu gewährleisten, dadurch, dass es ein Mittel zur Befestigung im Fahrgastraum eines Fahrzeugs mittels einer Basis (2) zur Befestigung an einem festen Teil eines Fahrzeugs durch eine instabile Verbindung aufweist, die dazu ausgelegt ist, eine oszillierende Bewegung des Kartuschenhalters um eine Referenzposition bei Beschleunigungen des festen Teils des Fahrzeugs entlang mindestens einer Achse zu gewährleisten, und dadurch, dass der Kartuschenhalter über eine flexible Stange (3), die an ihrem der Kartusche (1) nächstgelegenen Ende mit einem Gewicht (7) versehen ist, mit der Basis verbunden ist.

2. Duftverteilungssystem nach Anspruch 1, **dadurch gekennzeichnet, dass** die Kartusche (1) über der Basis (2) angeordnet ist.

3. Duftverteilungssystem nach Anspruch 1 oder 2, **dadurch gekennzeichnet, dass** die oszillierenden Bewegungen einen Vektor senkrecht zur Oberfläche des Gitters (5) umfassen.

## Claims

1. A fragrance diffusion system comprising at least one cartridge (1) containing solid elements loaded by adsorption with an olfactory composition and opening through a grid (5) having orifices with a section smaller than the section of said solid elements, **characterised in that** said solid elements consist of spheroid beads made of a polymer material, **in that** said cartridge (1) is mounted on a cartridge support holding the cartridge and connected to a base (2) for fastening on a fixed portion by an unstable connection capable of ensuring an oscillatory movement of said cartridge support around a reference position, **in that** it has a means for fastening in the passenger compartment of a vehicle via a base (2) for fastening on a fixed portion of a vehicle by an unstable connection capable of ensuring an oscillatory movement of said cartridge support around a reference position during the accelerations of said fixed portion of the vehicle according to at least one axis, and **in that** said cartridge support is connected to the base by a flexible rod (3) provided with a mass (7) at its end the closest to the cartridge (1).

2. The fragrance diffusion system according to claim 1, **characterised in that** said cartridge (1) is arranged above said base (2).

3. The fragrance diffusion system according to claim 1 or 2, **characterised in that** said oscillatory movements include a vector perpendicular to the surface of said grid (5) .
